(19) Europäisches Patentamt
European Patent Office
Office européen des brevets

(11) Numéro de publication: **0 124 439**
**B1**

# (12) FASCICULE DE BREVET EUROPEEN

(45) Date de publication du fascicule du brevet: **07.01.87**

(51) Int. Cl.[4]: **C 12 P 19/04**, C 08 B 37/06

(21) Numéro de dépôt: **84400845.8**

(22) Date de dépôt: **25.04.84**

(54) **Procédé de modification des pectines de betterave, produits obtenus et leurs applications.**

(30) Priorité: **29.04.83 FR 8307208**

(43) Date de publication de la demande:
**07.11.84 Bulletin 84/45**

(45) Mention de la délivrance du brevet:
**07.01.87 Bulletin 87/2**

(84) Etats contractants désignés:
**BE DE FR GB IT LU NL**

(56) *Documents cités:*
**CH - A - 244 249**

**LEBENSMITTEL - WISSENSCHAFT UND TECHNOLOGIE, vol. 8, no. 3, 1975, pages 121-122; I.L. GATFIELD et al.: "Enzymatic reactions in the presence of polymers: influence of pH upon the interaction between horseradish peroxidase and ionic polymers"**
**CHEMICAL ABSTRACTS, vol. 81, 1974, page 122, no. 87153w, Columbus, Ohio, US; P.J. BRIGNAC Jr. et al.: "Oxidation of 2-naphthol, 1-naphthol, and 2,7-dihydroxynaphthalene by horseradish peroxidase and hydrogen peroxide"**

(73) Titulaire: **INSTITUT NATIONAL DE LA RECHERCHE AGRONOMIQUE, La Gérandière, F-44072 Nantes Cedex (FR)**

(72) Inventeur: **Rombouts, Franciscus Maria Inst. Nat. des Hautes, Etudes Agronomiques Dépt. des Scs Alimentaires, de Dreyen 12 Wageningen (NL)**
Inventeur: **Thibault, Jean François Lab. de Biochimie et Tech., des glucides Inra Nantes Rue de Géraudière, F-44072 Nantes (FR)**
Inventeur: **Mercier, Christiane Lab. de Biochimie et Tech., des glucides Inra Nantes rue de Géraudière, F-44072 Nantes Cedex (FR)**

(74) Mandataire: **Phélip, Bruno et al, c/o Cabinet Harlé & Phélip 21, rue de la Rochefoucauld, F-75009 Paris (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).



ACTORUM AG

**Description**

L'invention concerne un procédé de modification des pectines de betterave, les produits obtenus par ce procédé et leurs applications notamment dans les industries alimentaires, cosmétiques ou pharmaceutiques.

Les pectines sont des molécules naturelles constituées d'acide polygalacturonique partiellement méthylé, c'est-à-dire dans lesquelles les fonctions acides sont partiellement estérifiées du méthanol; le motif fondamental de ces polymères répond à la formule:

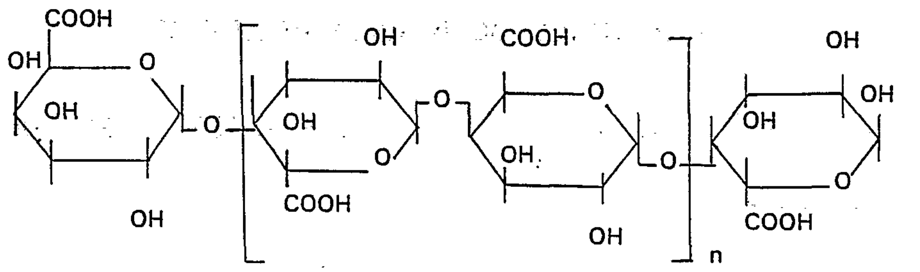

Certaines de ces pectines sont utilisées pour former des gels aqueux ou pour épaissir différents milieux. La possibilité et les conditions de formation de ces gels dépendent essentiellement de la structure de la pectine qui est elle-même fonction du végétal dont elle est extraite et du procédé d'extraction. Ainsi, on n'utilise actuellement que les pectines extraites des pommes et des agrumes pour préparer soit des gelées et confitures, soit des cosmétiques, ou encore des adjuvants épaississants et déshydratants de diverses compositions.

On sait que les pectines dites fortement méthylées, c'est-à-dire celles dont plus de 50% des groupes carboxyliques des acides galacturoniques sont estérifiés par le méthanol, gélifient en présence de saccharose et en milieu acide; aussi elles sont particulièrement utilisées à la confection des gelées et des confitures.

Les pectines moins méthylées sont gélifiées en présence d'ions $Ca^{2+}$, grâce à la formation de liaisons de coordinence entre les atomes d'oxygène et le cation; la préparation de gels convenables est toutefois délicate du fait de phénomènes secondaires de précipitation, notamment lors de l'addition de la solution de $Ca^{2+}$ aux pectines.

Certaines pectines naturelles, vraisemblablement parce qu'elles portent des substituants sur la chaîne polygalacturonique, ne forment pas de gels lorsqu'on leur applique les procédés connus de gélatification c'est-à-dire soit une addition de saccharose en milieu acide, soit une addition de $Ca^{2+}$.

Ces informations sur la gélification des pectines figurent notamment dans l'ouvrage «Les polymères végétaux, polymères pariétaux et alimentaires non azotés». Bernard MONTIES et Claude COSTES - Editions Gauthier-Villars (1980) pp. 232-251.

On sait que les pectines extraites del pulpes de betterave ne forment pas de gels convenables par les procédés précédemment rappelés; on pense que la présence de groupes acétyles, résultant de l'esterification de certaines groupes hydroxyles, jouerait un rôle dans cette absence de gélification; comme ces polymères ont des masses moléculaires relativement faibles, ils ne peuvent être par ailleurs utilisés comme agents épaississants. C'est pourquoi aucune utilisation industrielle n'est connue actuellement, malgré l'abondance de cette matière première: la pectine constitue 15 à 20% de la matière sèche des marcs de betterave, comme elle constitue 15 à 20% de celle des marcs de pomme.

Ainsi, le procédé selon l'invention, qui permet d'utiliser ces pectines après leur modification, présente un grand intérêt économique, puisqu'il permet la valorisation des résidus issus des betteraves, notamment sucrières.

L'invention concerne un procédé de traitement des pectines de betterave qui consiste à effectuer une réticulation oxydative enzymatique des pectines, ce qui conduit à des produits ayant des masses moléculaires plus élevées, qui seront solubles dans l'eau ou donneront des gels avec l'eau. La réticulation est réalisée en faisant réagir un système oxydant, comprenant un agent oxydant, qui est le peroxyde d'hydrogène et une enzyme ayant ledit agent oxydant comme substrat et qui consiste en un peroxydase. L'invention concerne aussi les pectines modifiées obtenues par ce procédé de réticulation, les gels aqueux résultant de la mise en œuvre de procédé, ainsi que les applications de ces pectines modifiées et de ces gels comme agents épaississants et gélifiants, notamment dans les industries alimentaires, cosmétiques ou pharmaceutiques.

Le document CH-A-244 249 décrit un procédé de dégradation oxydative de pectines au moyen de peroxyde d'hydrogène, dans la publication «Lebensmittel Wissenschaft und Technologie, vol. 8 n° 3, 1975, pages 121-122, Ian L. Gatfield et al, on décrit l'action de différentes pectines sur l'activité peroxydasique. Ni le problème qui est à la base de la présente invention, ni sa solution n'ont été divulgués ou suggérés par cet état antérieur de le technique.

Le procédé de l'invention n'a jamais été utilisé pour traiter des pectines; la Demanderesse a d'ailleurs constaté qu'il ne peut être appliqué avec succès qu'à certaines pectines et que celles issue des marcs de pomme, des agrumes, cerises, d'abricots ou des pommes de terre ne réticulent pas dans ces conditions; il donne pourtant de bons résultats avec les pectines tirées des betteraves, notamment sucrières, quelle que soit leur provenance.

Les pectines sont extraites des pulpes et marcs de betterave selon des procédés connus en eux-mêmes et par exemple par traitement des pulpes par une solution aqueuse acide chaude pendant quelques

heures; ainsi, on peut mettre en contact pendant 1 à 2 heures les pulpes récupérées avec une solution diluée d'acide sulfurique dont le pH est 1,5 à 2, à une température de 80°C. Lorsqu'un tel procédé d'extraction aboutit à des solutions de pectines de concentration convenable, on peut effectuer directement la réticulation dans ce milieu après l'avoir mis à un pH convenant; autrement, les pectines sont précipitées dans leur milieu d'extraction selon des méthodes connues en elles-mêmes, puis isolées par filtration et séchées pour donner des poudres, qui seront ensuite traitées par le procédé selon l'invention.

Le procédé selon la présente invention comprend notamment les étapes consistant à:

a) ajouter à une solution aqueuse de pectine une quantité de peroxydase comprise entre 0,01 mg et 1 mg pour 1 g de pectine, puis

b) ajouter une solution aqueuse de peroxyde d'hydrogène contenant 10 µmole à 1 mmole de $H_2O_2$ pour 1 g de pectine, et

c) si nécessaire, ajouter 0,1 mg environ de catalase pour détruire l'excès de $H_2O_2$.

Selon une mise en œuvre préférée de l'invention, la réticulation enzymatique oxydative est réalisée par addition à la solution aqueuse de pectine de betterave de concentration choisie, de 0,3 à 0,6 mg de peroxydase par gramme de pectine et de 13 µmoles à 36 µmoles environ de peroxyde d'hydrogène en solution aqueuse par gramme de pectine. On effectue également la réticulation avec addition de 0,03 mg à 0,1 mg de peroxydase et 0,25 à 1,0 millimole de peroxyde d'hydrogène par gramme de pectine en solution; dans ce cas, l'excès de peroxyde est détruit par addition d'une autre enzyme, la catalase. La masse moléculaire des pectines modifiées obtenues est fonction des concentrations et des proportions relatives des réactifs.

Les solutions diluées de peroxyde d'hydrogène étant peu stables, on prépare de préférence le réactif extemporanément à partir des solutions commerciales de $H_2O_2$ à 30%; les diluitions utilisées ont par exemple de 0,01 à 1% de $H_2O_2$ dans $H_2O$ (volume à volume).

La peroxydase peut être d'origine animale ou végétale, on utilise avantageusement la peroxydase radis noir type I, vendue par SIGMA (USA); elle peut être soit dissoute dans la solution aqueuse, soit immobilisée sur un support insoluble qui sera mis en contact avec le milieu réactionnel selon une manière connue en soi pour les réactions enzymatiques.

Le pH des solutions aqueuses de pectine peut être compris entre 4 et 7 environ; il est évidemment choisi de façon telle que l'enzyme du système oxydant puisse fonctionner et que les pectines ne soient pas dégradées.

Selon la concentration de la solution aqueuse en pectine de betterave, la nature de la pectine et la quantité de système oxydant, il se forme à température ambiante, en quelques minutes, après l'addition du système oxydant, soit un gel, soit une solution de viscosité supérieure à celle de la solution de départ contenant la pectine réticulée.

Selon un autre aspect de l'invention, on déterminera préalablement à la réticulation, quelle est la concentration convenable de la solution de pectine de départ pour obtenir, soit une solution de viscosité accrue, soit directement un gel. On a trouvé qu'en général avec des pectines de betterave ayant une masse moléculaire viscosimétrique de 40 000 à 50 000, un degré d'acétylation de 30 à 35% et dont la teneur en résidus de sucres neutres est comprise entre 15 et 20%, les solutions aqueuses dont la concentration en pectine est inférieure à 1,5 g/100 ml ne sont pas gélifiables et que, par contre, les solutions aqueuses de concentration supérieure à 2 g/100 ml donnent des gels épais, pour de faibles quantités de système oxydant; avec un net excès de $H_2O_2$ et d'enzyme, on obtient des gels pour des concentrations en pectine aussi faibles que 0,3 g/100 ml. En général, les solutions de pectine de betterave ont une concentration maximale de 4 g/100 ml, étant donné leur solubilité aqueuse limitée.

Les nouvelles pectines modifiées selon l'invention peuvent être isolées des milieux aqueux où elles ont été préparées; par exemple elles sont précipitées par addition d'un solvant à la phase aqueuse, tels que l'alcool ou l'acétone, ou par addition de sels minéraux (relargage); on isole ensuite les précipités par filtration ou centrifugation et on les sèche à pression ordinaire ou sous vide avec chauffage modéré, vers 40°C. On ajoute en général au moins un volume de solvant de précipitation par rapport au volume de la solution aqueuse et de préférence environ 4 volumes.

La possibilité d'isoler les pectines gélifiées sous forme de poudre sèche est un autre avantage de l'invention; les gels de pectines de pomme ou agrume précédemment connus ne possédaient pas cette propriété. La poudre issue des gels selon l'invention, remise en présence de la quantité convenable d'eau, redonne un gel.

Les poudres de pectines modifiées obtenues selon le procédé de l'invention peuvent être utilisées comme agents épaississants ou gélifiants; elles sont très hydrophiles et sont capables de reprendre de grandes quantités d'eau.

En particulier, ces pectines modifiées sont utilisables pour préparer des produits alimentaires, tels que des desserts lactés, et par exemple des flans préparables à température ambiante. Elles entreront aussi avantageusement dans la composition de dessert glacés; on a en effet montré que les gels selon l'invention sont parfaitement stables si on leur fait subir des cycles de congélation/décongélation de —20°C à la température ambiante.

Une autre application avantageuse des pectines selon l'invention est la préparation de pommades, qui seront utilisées dans les domaines cosmétiques ou pharmaceutiques; les pectines jouant le rôle d'excipients.

Selon une autre mise en œuvre de l'invention, on effectue la gélification de la solution aqueuse contenant les pectines de betterave en présence de divers principes actifs, ce qui aura pour résultat la formation de produits particulièrement homogènes ou stabilisés. Ceci sera appliqué, par exemple, pour la microencapsulation d'arômes, de composés ayant une activité pharmacologique ou autre, ou encore

pour la détoxification de certains milieux en emprisonnant, par exemple, des métaux lourds.

Dans ce qui suit, on donnera des exemples de réalisation de l'invention.

*Exemple 1*

Préparation d'une solution de pectines modifiées:

A 10 ml d'une solution aqueuse de pectine de betterave ayant un pH de 6 et contenant 0,3 mg de peroxydase de radis noir par gramme de pectine, on ajoute 1 ml d'une solution aqueuse de peroxyde d'hydrogène de telle façon que l'on ait 13 µmoles d'$H_2O_2$ par gramme de pectine. La réticulation est terminée après quelques minutes d'agitation à température ambiante.

Ce procédé a été appliqué a des solutions de différentes concentrations en pectines et la viscosité des solutions obtenues mesurées. Dans le tableau I figurent les résultats des essais exprimés en viscosités intrinsèques et en masses moléculaires, calculés selon la méthode décrite par H.S. OWENS, H. LOTZKAR, T.H. SCHULTZ et W.D. MACLAY dans J. Am. Chem. Soc. 68 1628-1632 (1946).

TABLEAU I

| Concentration en pectine (g/100 ml) | Viscosité intrinsèque ($cm^3/g$) | Masse moléculaire |
|---|---|---|
| témoin * | 248 | 46 000 |
| 0,6 | 435 | 70 000 |
| 0,9 | 468 | 73 900 |
| 1,5 | 461 | 73 000 |

* solution aqueuse de pectine, sans addition du système oxydant réticulant.

*Exemple 2*

Gélification d'une solution aqueuse de pectines de betterave:

On introduit dans 1 ml d'une solution de pectine de betterave (de concentration 3 g/100 ml), à pH 6 environ, 0,1 ml d'une solution aqueuse de peroxydase (de concentration 0,1 mg/ml) et 0,1 ml d'une solution aqueuse de peroxyde d'hydrogène de concentration 4,3 µmoles par ml; on agite mécaniquement pendant 1 à 2 minutes jusqu'à gélification. Le gel est translucide et reste stable, durant plusieurs mois à la température ambiante.

Dans le tableau II figurent les caractéristiques rhéologiques d'un gel préparé selon ce procédé et celles d'un gel de pectine amidée préparé selon la méthode décrite par S.A. BLACK et C.J.B. SMIT dans J. Food Science 37 726-29 (1972) à titre de comparaison.

TABLEAU II

| gel | Module «apparent» de rigidité g/cm | Force à la rupture (g) | Déformation (cm) |
|---|---|---|---|
| pectine amidée | 15,2 | 11,80 | 0,45 |
| selon ex. 2 | 11,6 | 36,3 | 0,67 |

Les mesures ont été effectuées avec un appareil INSTRON ®.

Ces résultats montrent que le gel selon l'invention est moins «cassant» que le gel préparé selon une méthode connue.

*Exemple 3*

Préparation d'une poudre de pectines modifiées:

Un gel de pectine de betterave est préparé comme décrit dans l'exemple 2. On y ajoute 4 volumes d'éthanol aqueux (à 96%) sous agitation; la pectine modifiée qui précipite est isolée par filtration, puis séchée à température ambiante.

Le pouvoir hydrophile de cette poudre a été déterminé en mettant de la poudre en présence d'un excès d'eau, dans un récipient gradué, et en mesurant le volume du gel reformé à l'équilibre.

On a obtenu 110 ml de gel avec 1 g de poudre de pectine de betterave préparée comme décrit cidessus.

*Exemple 4*

En utilisant le même mode opératoire que celui décrit à l'exemple 2 et faisant varier les proportions relatives des réactifs, on obtient les résultats du tableau III.

TABLEAU III

| concentration en pectine (g/l) | concentration en peroxydase (mg/l) | concentration en $H_2O_2$ (mmole/l) | gélification | augmentation de la masse moléculaire |
|---|---|---|---|---|
| 6,26 | 0,25 | 2 | + | |
| 5,01 | 0,25 | 2 | + | |
| 3,76 | 0,25 | 2 | — | |
| 2,51 | 0,25 | 2 | — | |
| 5,45 | 0,67 | 2 | — | |
| 5,61 | 0,1 | 2 | — | 140% |
| 3,48 | 1,7 | 8,3 | + | |
| 3,5 | 2,0 | 2 | + | |
| 3,01 | 8,3 | 4 | — | 150% |
| 4,88 | 0,07 | 0,7 | — | 210% |
| 3,19 | 0,04 | 1,3 | — | 180% |

**Revendications**

Procédé de modification des pectines de betterave, caractérisé par le fait qu'on fait réagir, sur lesdites pectines, un système oxydant comprenant, d'une part, le peroxyde d'hydrogène comme agent oxydant et, d'autre part, une peroxydase dont les substrat est constitué par ledit agent oxydant.

2. Procédé selon la revendication 1, caractérisé par le fait qu'il comprend les étapes consistant à:

(a) ajouter à une solution aqueuse de pectine une quantité de peroxydase comprise entre 0,01 mg et 1 mg pour 1 g de pectine; puis

(b) ajouter une solution aqueuse de peroxyde d'hydrogène contenant de 10 µmole à 1 mmole de $H_2O_2$ pour 1 g de pectine; et

(c) si nécessaire, ajouter 0,1 mg environ de catalase pour détruire l'excès de $H_2O_2$.

3. Procédé selon la revendication 2, dans lequel on isole la pectine modifiée à l'état de poudre, caractérisé par le fait qu'il comprend les étapes consistant à:

— ajouter au milieu réactionnel, après la fin de l'oxydation, un agent de relargage choisi parmi les liquides comme un alcool ou une cétone, et les sels minéraux neutres;

— filtrer ensuite le précipité formé et le sécher.

4. Pectine de betterave modifiée hydrosoluble préparée par le procédé tel que défini à l'une des revendications 1 à 3, dans lequel la concentration de la solution de pectine de départ et les quantités de système oxydant sont trop faibles pour entraîner la gélification du milieu réactionnel.

5. Pectine modifiée selon la revendication 4, préparée par le procédé tel que défini à l'une des revendications 2 et 3, dans lequel la solution de pectine de départ est de concentration inférieure à 1,5 g/100 ml et le système oxydant comporte de 0,3 à 0,6 mg de peroxydase et de 13 à 36 µmoles de $H_2O_2$ pour 1 g de pectine.

6. Pectine de betterave modifiée ayant un pouvoir gélifiant en milieu aqueux, préparée par le procédé tel que défini à l'une des revendications 1 à 3, dans lequel la concentration de la solution de pectine de départ et les quantités de système oxydant sont suffisantes pour qu'il y ait gélification du milieu réactionnel.

7. Pectine modifiée selon la revendication 6, préparée par le procédé tel que défini à l'une des revendications 2 et 3, dans lequel la solution de pectine de départ est de concentration supérieure à 2 g/ml et le système oxydant comporte de 0,3 à 0,6 mg de peroxydase et 13 à 36 µmoles de $H_2O_2$ pour 1 g de pectine.

8. Pectine modifiée selon la revendication 6, préparée par le procédé tel que défini à l'une des revendications 2 et 3, dans lequel la solution de pectine de départ est de concentration supérieure à 0,3 mg/100 ml et le système oxydant comporte de 0,03 à 0,1 mg de peroxydase et de 0,25 à 0,1 mmole de $H_2O_2$ pour 1 g de pectine.

9. Gels aqueux, caractérisés par le fait qu'ils sont préparés par mise au contact de l'eau d'une pectine modifiée en poudre telle que définie à l'une des revendications 6 à 8.

10. Application d'une pectine modifiée telle que définie à l'une des revendications 4 à 8, comme agent épaississant ou gélifiant de produits alimentaires, de produits cosmétiques ou de produits pharmaceutiques.

**Pantentansprüche**

1. Verfahren zur Modifikation von Rübenpektinen, dadurch gekennzeichnet, dass man auf die genannten Pektine ein oxidierendes System einwirken lässt, das einerseits Wasserstoffperoxid als Oxidationsmittel und andererseits eine Peroxidase, deren Substrat aus dem genannten Oxidationsmittel besteht, enthält.

2. Verfahren nach Anspruch 1, dadurch gekennzeichnet, dass es die folgenden Stufen umfasst:

(a) Zugeben zu einer wässrigen Pektinlösung einer Menge der Peroxidase zwischen 0,01 mg und 1 mg pro 1 g Pektin, dann

(b) Zugeben einer wässrigen Wasserstoffperoxidlösung, die 10 µmol bis 1 mmol $H_2O_2$ pro 1 g Pektin enthält, und

(c) wenn nötig, Zugeben von ungefähr 0,1 mg Katalase, um den Überschuss an $H_2O_2$ zu zerstören.

3. Verfahren nach Anspruch 2, in dem man das modifizierte Pektin in pulverförmigem Zustand isoliert, dadurch gekennzeichnet, dass es die folgenden Stufen umfasst:

— Zugabe eines Aussalzmittels, ausgewählt aus Flüssigkeiten wie einem Ankohol oder einem Keton und aus neutralen Mineralsalzen zum Reaktionsmedium nach dem Ende der Oxidation,

— anschliessendes Abfiltrieren des gebildeten Niederschlags und Trocknung.

4. Modifiziertes wasserlösliches Rübenpektin, hergestellt durch das Verfahren wie es in einem der Ansprüche 1 bis 3 definiert ist, bei dem die Konzentration der Ausgangspektinlösung und die Mengen des oxidierenden Systems zu schwach sind, um die Gelbildung des Reaktionsmilieus zur Folge zu haben.

5. Modifiziertes Pektin nach Anspruch 4, hergestellt durch das Verfahren wie es in einem der Ansprüche 2 und 3 definiert ist, in dem die Lösung des Ausgangspektins eine Konzentration unterhalb 1,5 g/100 ml aufweist und das Oxidationssystem 0,3 bis 0,6 mg Peroxidase und 13 bis 36 µmol $H_2O_2$ pro 1 g Pektin umfasst.

6. Modifiziertes Rübenpektin mit Gelfähigkeit im wässrigen Milieu, hergestellt nach dem Verfahren wie es in einem der Ansprüche 1 bis 3 definiert ist, in dem die Konzentration der Ausgangspektinlösung und die Mengen des Oxidationssystems ausreichend sind, damit die Gelbildung des Reaktionsmilieus stattfindet.

7. Modifiziertes Pektin nach Anspruch 6, hergestellt nach dem Verfahren wie es in einem der Ansprüche 2 und 3 definiert ist, in dem die Lösung des Ausgangspektins eine Konzentration oberhalb 2 g/ml aufweist und das Oxidationssystem 0,3 bis 0,6 mg Peroxidase und 13 bis 36 µmol $H_2O_2$ pro 1 g Pektin umfasst.

8. Modifiziertes Pektin nach Anspruch 6, hergestellt nach dem Verfahren wie es in einem der Ansprüche 2 und 3 definiert ist, in dem die Ausgangspektinlösung eine Konzentration über 0,3 mg pro 100 ml aufweist und das Oxidationssystem 0,03 bis 0,1 mg Peroxidase und 0,25 bis 0,1 mmol $H_2O_2$ pro 1 g Pektin umfasst.

9. Wässrige Gele, dadurch gekennzeichnet, dass sie hergestellt worden sind, indem man ein modifiziertes pulvriges Pektin, wie es in einem der Ansprüche 6 bis 8 definiert ist, mit Wasser in Kontakt bringt.

10. Anwendung eines modifizierten Pektins, wie es in einem der Ansprüche 4 bis 8 definiert ist, als Verdickungsmittel oder Gelbildungsmittel in Nahrungsmitteln, Kosmetika oder Pharmazeutika.

**Claims**

1. Process for the modification of beet pectins,

characterized in that an oxidizing system comprising hydrogen peroxide as oxidizing agent on the one hand and, a peroxydase, the substrate of which consists of the said oxidizing agent on the other, is reacted with the said pectins.

2. Process according to Claim 1, characterized in that it comprises steps consisting in:

(a) adding a quantity of peroxydase of between 0,01 mg and 1 mg for 1 g of pectin to an aqueous solution of pectin; followed by

(b) adding an aqueous solution of hydrogen peroxide containing from 10 µmol to 1 mmol of $H_2O_2$ for 1 g of pectin; and

(c) if required, adding approximately 0,1 mg of catalase in order to destroy the excess of $H_2O_2$.

3. Process according to claim 2, in which the modified pectin is isolated in the form of a powder, characterized in that it comprises steps consisting in: adding a draining out agent chosen from liquids such as an alcohol or a ketone, and neutral inorganic salts to the reaction medium at the end of oxidation; then filtering the precipitate formed and drying it.

4. Water-soluble modified beet pectin prepared by the process as defined in one of Claims 1 to 3, in which the concentration of the starting pectin solution and the quantities of the oxidizing system are too small to produce the gelling of the reaction medium.

5. Modified pectin according to Claim 4, prepared by the process as defined in one of Claims 2 and 3, in which the starting pectin solution is of a concentration less than 1,5 g/100 ml and the oxidizing system comprises from 0,3 to 0,6 mg of peroxydase and from 13 to 36 µmol of $H_2O_2$ for 1 g of pectin.

6. Modified beet pectin which has a gelling power in an aqueous medium, prepared by the process as defined in one of Claims 1 to 3, in which the concentration of the starting pectin solution and the quantities of the oxidizing system are sufficient for the gelling of the reaction medium to take place.

7. Modified pectin according to Claim 6, prepared by the process as defined in one of Claims 2 and 3, in which the starting pectin solution is of a concentration greater than 2 g/ml and the oxidizing system contains from 0,3 to 0,6 mg of peroxydase and 13 to 36 µmols of $H_2O_2$ for 1 g of pectin.

8. Modified pectin according to Claim 6, prepared by the process as defined in one of Claims 2 and 3, in which the starting pectin solution is of a concentration greater than 0,3 mg/100 ml and the oxidizing system contains from 0,03 to 0,1 mg of peroxydase and from 0,25 to 0,1 mmol of $H_2O_2$ for 1 g of pectin.

9. Aqueous gels, characterized in that they are prepared by bringing into contact with water a modified pectin powder as defined in one of Claims 6 to 8.

10. Application of a modified pectin as defined in one of Claims 4 to 8, as thickening or gelling agent for food products, cosmetic products or pharmaceutical products.